# EUROPEAN PATENT SPECIFICATION

(11) **EP 4 460 553 B1**
(45) Date of publication and mention of the grant of the patent: **30.07.2025**
(21) Application number: 22839730.3
(22) Date of filing: 16.12.2022
(51) Int. Cl.: C11D 1/62, C11D 3/20, C11D 3/48, A01N 33/12, A01N 31/16, A01P 3/00

(54) **AN ANTIFUNGAL COMPOSITION**
ANTIMYKOTISCHE ZUSAMMENSETZUNG
COMPOSITION ANTIFONGIQUE

(30) Priority: 04.01.2022 EP 22150129
(43) Date of publication of application: 13.11.2024
(73) Proprietor: Unilever IP Holdings B.V., 3013 AL Rotterdam (NL); Unilever Global IP Limited, Wirral, Merseyside CH62 4ZD (GB)
(72) Inventor: MAHAPATRA, Samiran, 6708 WH Wageningen (NL); MEDEPALLI, Srilaxmi Venkata, 6708 WH Wageningen (NL); MOHAPATRA, Namisha, 6708 WH Wageningen (NL); VARMA, Sandeep, 6708 WH Wageningen (NL)
(74) Representative: Corsten, Michael Allan
(86) International application number: PCT/EP2022/086369
(87) International publication number: WO 2023/131499

(56) References cited:
- WO-A1-2018/160104
- CN-A- 111 417 373
- GB-A- 2 368 591
- US-A1- 2006 134 239
- LIPNICKI B ED - LI RU: "THE ANTIFUNGAL PROPERTIES OF HEXYLRESORCINOL", vol. 11, 1 January 1963 (1963-01-01), pages 417 - 422, XP009536189, ISSN: 0004-069X, Retrieved from the Internet <URL:http://www.ncbi.nlm.nih.gov/pubmed/14059317>
- CHEN QING-XI ET AL: "Inhibitory Effects of Hexylresorcinol and Dodecylresorcinol on Mushroom (Agaricus bisporus) Tyrosinase", PROTEIN JOURNAL, vol. 23, no. 2, 2 February 2004 (2004-02-02), NL, pages 135 - 141, XP055926438, ISSN: 1572-3887, [retrieved on 20220531], DOI: 10.1023/B:JOPC.0000020080.21417.ff

## Description

### Field of the Invention

The present invention relates to use of an alkyl substituted dihydroxy benzene compound for inhibition of fungal melanin and for reducing adhesion of fungus on to surfaces. The invention more particularly relates to an antifungal composition for hard surface cleaning applications including toilet cleaning which is very effective against growth of fungus in addition to antibacterial efficacy. It has also been seen to be effective in fabric cleaning applications

### Background of the Invention

Disinfecting cleaning compositions are of great benefit to individuals, since proper use generally may reduce the number of germs and pathogens the individual is exposed to. Such compositions may for instance play an important role in reducing the occurrence and spread of infectious diseases. Disinfecting compositions comprising chlorine-based bleaches are known. Such compositions are sometimes considered to be rather harsh as it may exhibit undesirable side effects, such as corrosion, malodour (like a chlorine smell) and skin irritation or skin-sensitising effects when it comes in contact with a person's skin or mucous membrane. Therefore, many consumers prefer using compositions containing milder bleaches e.g. peroxide compounds.

Such compositions are used for cleaning hard surfaces e.g. cleaning of floors, table tops, kitchen surfaces and utensils. They are also used for cleaning bathrooms and toilets. The compositions may also include certain antimicrobial agents like a cationic surfactant which delivers the desired antimicrobial efficacy in addition to helping clean surfaces to be free of dirt and oils as it is also a surfactant.

Washing fabrics, also known as laundering or simply as laundry, makes up one of the main chores that people undertake routinely. For this, people have been relying on detergent compositions that are widely available. During day-to-day usage of fabrics, particles that may be present in the air e.g. pollen grains, dust and dirt tend to settle on a fabric. In addition, germs or microbes e.g. bacteria, fungi, spores and viruses, that are present in the air, also tend to settle on a fabric just like they tend to settle on other surfaces e.g. floors. These microbes tend to thrive when they come in to contact with substances like sweat and sebum. As a result, microbial growth and/or microbial secretions, lead to problems e.g. leaving behind stains or spots which tend to affect appearance of a fabric, creation of unhygienic conditions due to thriving germs; and at times, generation of malodour. Further, certain fabric especially those used in cleaning surfaces like mops, towels etc tend to develop an unpleasant blackish or grey colouration which is due fungal growth on such surfaces.

The present inventors noticed that compositions known in the art are good in delivering desired anti-bacterial efficacy but lack in delivering antifungal efficacy. A representative fungus which is difficult to remove from hard surfaces in the homes is *Aspergillus Brasiliensis.* The present inventors therefore took it upon themselves to solve the above problem. They are aware that many antifungal actives are available which ensure that the fungus is killed or deactivated. However, such actives could be harsh and may have to be used in large concentrations to achieve the desired efficacy. They were looking for milder solutions to this problem. They took a different approach which does not necessarily cause killing of the fungus. They looked for actives that target the fungal melanin thereby affecting the integrity of the fungal cell. Once this is achieved, they hypothesized that this will weaken the adhesion of the fungus to the surface thereby making it easy to rinse off, scrape off or remove the fungus from the surface either with water or a mild surfactant solution. To their utter surprise they found that certain alkyl substituted dihydroxy benzene compounds which are also known as alkyl resorcinols are capable of inhibiting formation of fungal melanin through the DHN melanin pathway. In other words, they found that alkyl substituted dihydroxy benzene is capable of inhibiting fungi that produce DHN melanin. Such pathway is found in fungus of interest like Aspergillus *brasiliensis, Aspergillus nidulans, Aspergillus niger, Alternaria alternata, Cladosporium carionii, Exophiala jeanselmei, Fonsecaea compacta, Fonsecaea pedrosoi, Hendersonula toruloidii, Phaeoannellomyces wernickii, Phialophora richardsiae, Phialophora verrucosa, Wangiella dermatitidis, and Xylohypha. Bantiana.* Of more specific interest are fungi of the Aspergillus genus, most specifically Aspergillus *brasiliensis.*

DHN melanin is not produced in mushroom. Mushroom generally produce DOPA melanin through the tyrosinase pathway.

, Melanin is a molecule that gives colour e.g. grey, brown or black colour to the fungus adhering on the surfaces. While on the one hand it gives a cosmetic benefit by ensuring that the fungi adhering to the surface is made colourless thereby giving an appearance of cleaning, they were even more surprised to see a significantly better benefit. They found that fungi so treated with the alkyl resorcinols could also be easily removed from such surfaces through use of simple cationic surfactants, without any significant manual scrubbing. This could be easily used by consumers for cleaning by either using a composition comprising alkyl resorcinol and a cationic surfactant; or by a method (using a kit) comprising first applying the alkyl resorcinol to the surface followed by cleaning the surface with a cleaning composition. To the knowledge of the present inventors such a composition or method is not known or published in the art.

It is an object of the present invention to provide for inhibition of fungal melanin.

It is another object of the present invention to provide for a composition which is efficacious in removal of fungus from surfaces. US2006134239A1 relates to a method for reducing the adhesion of microorganism comprising contacting the surface with at least one of patchouli oil. GB2368591A relates to an aqueous hard surface cleaning and disinfecting composition comprising at least one cationic surfactant having germicidal properties.

### Summary of the Invention

According to the first aspect of the present invention there is provided use of an alkyl substituted dihydroxy benzene compound with the alkyl group having 2 to 6 carbon atoms for inhibition of fungal melanin in fungi that produce DHN melanin.

Another aspect of the present invention provides for use of an alkyl substituted dihydroxy benzene compound with the alkyl group having 2 to 6 carbon atoms for reducing adhesion of fungus on surfaces. Such fungi are those that produce DHN melanin.

Yet another aspect of the present invention provides for a composition for removal of fungus from surfaces comprising
(i) alkyl substituted dihydroxy benzene wherein the alkyl group comprises 2 to 6 carbon atoms; and
(ii) one or more cationic surfactant selected from
   alkyl dimethyl benzylammonium chloride(ADBAC),
   alkyl diethyl benzylammonium chloride (ADEBAC), bis (C8-C18) alkyl di methyl quaternary ammonium chloride preferably didecyl dimethyl ammonium chloride (DDAC), cetyl trimethyl ammonium chloride (CTAC), cetyl pyridinium chloride (CPC), cetrimonium bromide , benzethonium chloride (BZE); and dimethyldioctadecylammonium chloride.

### Detailed Description of the Invention

For the avoidance of doubt, any feature of one aspect of the present invention may be utilised in any other aspect of the invention. The word "comprising" is intended to mean "including" but not necessarily "consisting of" or "composed of". Thus, the term "comprising" is meant not to be limiting to any subsequently stated elements, but rather to optionally also encompass non-specified elements of major or minor functional importance. In other words, the listed steps or options need not be exhaustive. Whenever the words "including" or "having" are used, these terms are meant to be equivalent to "comprising" as defined above. It is noted that the examples given in the description below are intended to clarify the invention and are not intended to limit the invention to those examples per se. Except in the examples, or where otherwise explicitly indicated, all numbers in this description indicating amounts of material or conditions of reaction, physical properties of materials and/or use are to be understood as modified by the word "about". Unless specified otherwise, numerical ranges expressed in the format "x to y" are understood to include x and y. When for a specific feature multiple preferred ranges are described in the format "x to y", it is understood that all ranges combining the different endpoints are also contemplated. Unless specified otherwise, amounts as used herein are expressed in percentage by weight based on total weight of the composition and is abbreviated as "wt%".

The compositions of the present invention are preferred for non-therapeutic use, and more particularly preferred for use in cleaning surfaces, preferably wherein the surface is a hard surface and more preferably for use in cleaning applications such as cleaning kitchen, bathroom and floor surfaces and certain items in the kitchen like utensils. They may also be used for cleaning fabrics.

The present invention is particularly useful for inhibiting melanin in fungi that are capable of producing DHN melanin. This is especially useful to reducing adhesion of such fungi on surfaces so that they can be easily removed from those surfaces with mild scraping in presence of water or a mild surfactant solution. The inhibition of melanin in such fungi does not necessarily cause killing of the fungi. The fungi of interest are selected from one or more of Aspergillus *Brasiliensis, Aspergillus nidulans, Aspergillus niger, Alternaria alternata, Cladosporium carionii, Exophiala jeanselmei, Fonsecaea compacta, Fonsecaea pedrosoi, Hendersonula toruloidii, Phaeoannellomyces wernickii, Phialophora richardsiae, Phialophora verrucosa, Wangiella dermatitidis, and Xylohypha. Bantiana.* Preferably the fungi are of the Aspergillus genus, most specifically *Aspergillus Brasiliensis.*

The composition of the invention comprises alkyl substituted dihydroxy benzene wherein the alkyl group comprises 2 to 6 carbon atoms.

Alkyl substituted dihydroxy benzene has the general chemical structure as given below:

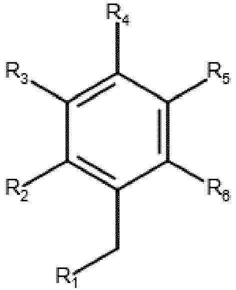

Wherein R₁ is C1 to C5 alkyl group; and each of R₂ to R₆ is either an OH group or H; and wherein at least two of R₂ to R₆ is OH.

Structure of more preferred alkyl substituted dihydroxy benzene for use in the present invention is

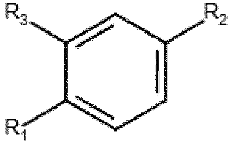

Wherein R₁ is a C₂ to C₆ alkyl group; R₂ and R₃ are OH

Preferably R₁ is an alkyl group comprising 2, 4 or 6 carbon atoms. The two hydroxy groups are generally at the 1 and 3 position. Such compounds are often referred to as substituted resorcinols. Thus, the most preferred compounds are ethyl resorcinol, butyl resorcinol or hexyl resorcinol. The 4-substituted resorcinol is most preferred. Thus the resorcinols which may be used are 4-ethyl resorcinol, 4-butyl resorcinol or 4-hexyl resorcinol. The more preferred compound for use in the present invention is one or both of 4-ethyl resorcinol and 4-hexyl resorcinol, most preferred being 4-ethyl resorcinol (4-ER). 4-ER is also known by its synonyms 4-ethylbenzene-1,3-diol, and 4-ethyl-1,3-benzenediol.

The composition preferably comprises 0.05 to 4% alkyl substituted dihydroxy benzene by weight of the composition. It is more preferably present in in 0.075 to 3.5 wt%.

The composition of the invention includes a cationic surfactant which is preferably a quaternary ammonium surfactant. Preferably the quaternary ammonium surfactant is selected from one or more of alkyl dimethyl benzylammonium chloride (ADBAC) which is also known as benzalkonium chloride (BKC), bis (C8-C18) alkyl di methyl quaternary ammonium chloride preferably didecyl dimethyl ammonium chloride (DDAC), cetyl trimethyl ammonium chloride (CTAC), cetyl pyridinium chloride (CPC), cetrimonium bromide, benzethonium chloride (BZE); and dimethyldioctadecylammonium chloride. Preferred aspect of the invention provides for the cationic surfactant to be chosen from one or more of BKC or DDAC, preferably BKC.

They are available as a single quaternary ammonium compound, as well as mixtures of two or more different quaternary ammonium compounds e.g. under trademarks BARDAC^{™}, BARQUAT^{®} and HYAMINE^{®} (all by Lonza); and BTC^{®} (by Stepan). Quaternary ammonium compounds available as a single quaternary ammonium compound include didecyl dimethyl ammonium chloride (available as BARDAC^{™} 2250 R and BTC^{®} 1010, both 50% active; and BARDAC^{™} 2280 R and BTC^{®} 1010-80, both 80% active), alkyl dimethyl benzyl ammonium chloride (available as BARQUAT^{®} MB-50, BARQUAT^{®} MX-50, BARQUAT^{®} OJ-50, HYAMINE^{®} 3500, BTC^{®} 50, BTC^{®} 50E, BTC^{®} 65, BTC^{®} 776, BTC^{®} 824, BTC^{®} 835; each 50% active; and the same is available as BARQUAT^{®} MB-80, BARQUAT^{®} MX-80, HYAMINE^{®} 3500-80, BTC^{®} 8248, BTC^{®} 8358; each 80% active

The cationic surfactant is preferably included in 0.01 to 2% by weight of the composition, more preferably in 0.1 to 1%, further more preferably in 0.25 to 0.75 % by weight of the composition.

The composition of the invention may be used in very many cleaning products like those for general purpose or toilet cleaning, for cleaning fabrics through fabric conditioners and through dishwash compositions. Such compositions comprise one or more surfactants in addition to the cationic surfactant used in the first aspect of the invention.

### General purpose or toilet cleaners

The composition of the invention may be formulated as a general purpose cleaner which is usually used to clean floors (in which case it may be known as a floor cleaner) or used to clean toilets (in which case it may be known as a toilet cleaner). It may also be used to clean other hard surfaces like furniture, table tops, kitchen platforms and other surfaces in homes, offices, restaurants and other public places.

The composition of the invention delivered as general purpose cleaner may further comprise a nonionic surfactant. Typically these can be included for the purpose of stabilising the compositions. Suitable nonionic surfactants include addition products of ethylene oxide and/or propylene oxide with fatty alcohols, fatty acids and fatty amines.

Preferably the nonionic surfactant has an HLB of from about 7 to about 20, more preferably from 10 to 18, e.g. 12 to 16. Genapol^{™} C200 (Clariant) based on coco chain and 20 EO groups is an example of a suitable nonionic surfactant.

If present, the nonionic surfactant is present in an amount from 0.1 to 3 wt.%, more preferably 0.15 to 0.4 wt.%, based on the total weight of the composition.

Ingredients like amphoteric surfactant and sequestrant may be included in the floor cleaner and toilet cleaner composition of the present invention. General purpose cleaners and toilet cleaners may be diluted before use. When diluted, they may be diluted with water in a weight ratio in the range of 1:10 to 1:1000.

### Fabric conditioning composition

The composition of the invention may also be delivered to provide the benefits of the invention to clothes or fabric that are laundered. It is preferably delivered through a fabric conditioning composition which additionally comprises 1 to 50% of a fabric softening active. Delivery through a fabric conditioning composition is preferred since it is included in the rinse stage (preferably the last rinse stage) of the laundering process. At this stage the fabric softening active ensures high levels of deposition of the bacteria on the fabric thereby ensuring long lasting hygiene benefit. The fabric conditioner composition is also referred to as a fabric softener. Fabric conditioners comprise active materials which soften or condition fabric. Examples of suitable fabric softening actives include: quaternary ammonium compounds, silicone polymers, polysaccharides, clays, amines, fatty esters, dispersible polyolefins, polymer latexes and mixtures thereof. The fabric softening active is preferably selected from a quaternary ammonium compound, or silicone polymer and mixtures thereof, more preferably a quaternary ammonium compound.

The fabric softening compounds may preferably be cationic or non-ionic. Preferably, the fabric softening compounds of the present invention are cationic.

Fabric conditioning compositions for use in accordance with the invention may be dilute or concentrated. Dilute products typically contain up to about 6 wt.% of the composition softening compounds, generally about 1 to 5 wt.%, whereas concentrated products may contain up to about 50 wt. % of the composition softening compounds, preferably from about 5 to about 50 wt.%, more preferably from 6 to 25 wt.%. Overall, the products of the invention may contain from 1 to 50 wt. %, preferably from 2 to 25 wt. % of the composition softening compounds, more preferably 2 to 20 wt. % of softening compounds.

The preferred softening compounds for use in fabric conditioner compositions of the invention are quaternary ammonium compounds (QAC). The QAC preferably comprises at least one chain derived from fatty acids, more preferably at least two chains derived from a fatty acids. Generally fatty acids are defined as aliphatic monocarboxylic acids having a chain of 4 to 28 carbons. Preferably the fatty acid chains are palm or tallow fatty acids. Preferably the fatty acid chains of the QAC comprise from 10 to 50 wt. % of saturated C18 chains and from 5 to 40 wt. % of monounsaturated C18 chains by weight of total fatty acid chains. In a further preferred embodiment, the fatty acid chains of the QAC comprise from 20 to 40 wt. %, preferably from 25 to 35 wt. % of saturated C18 chains and from 10 to 35 wt. %, preferably from 15 to 30 wt. % of monounsaturated C18 chains, by weight of total fatty acid chains.

The preferred quaternary ammonium fabric softening compounds for use in compositions of the present invention are so called "ester quats". Particularly preferred materials are the ester-linked triethanolamine (TEA) quaternary ammonium compounds comprising a mixture of mono-, di- and tri-ester linked components.

The composition of the invention delivered as fabric conditioning composition may further comprise a nonionic surfactant. Typically these can be included for the purpose of stabilising the compositions. Suitable nonionic surfactants are those described above as suitable for use in general purpose or toilet cleaning compositions. If present, the nonionic surfactant is present in an amount from 0.01 to 10 wt.%, more preferably 0.1 to 5 wt.%, based on the total weight of the composition. Thus, the fabric conditioning composition of the invention preferably comprises 0.1 to 5 wt% of a non-ionic surfactant preferably an fatty alcohol ethoxylate.

Co-softeners may be used in the fabric conditioning composition. When employed, they are typically present at from 0.1 to 20 wt.% and particularly at from 0.5 to 10 wt.%, based on the total weight of the composition. Preferred co-softeners include fatty esters, and fatty N-oxides. Fatty esters that may be employed include fatty monoesters, such as glycerol monostearate, fatty sugar esters, such as those disclosed WO 01/46361 (Unilever).

The compositions of the present invention may comprise a fatty complexing agent. Especially suitable fatty complexing agents include fatty alcohols and fatty acids. Of these, fatty alcohols are most preferred. Preferably the composition of the present invention comprises 0.5 to 20 wt.% perfume materials by weight of the composition, more preferably 1 to 15 wt.% perfume materials, most preferably 2 to 10 wt. % perfume materials.

The fabric conditioner composition is preferably in an aqueous form. The compositions preferably comprise from 75 to 95 wt.% water

The fabric conditioning composition may be used to treat fabrics either in a hand washing or a machine washing process. Preferably the fabric conditioner is used in the rinse stage of the washing process.

Preferably the clothes are treated with a 10 to 100 ml dose of fabric conditioner for a 4 to 7 kg load of clothes. More preferably, 10 to 80 ml for a 4 to 7 kg load of clothes.

The composition of the invention may also be delivered as a rim block. By a rim block is meant a shaped solid composition that is hung on to the rim of the toilet usually in a plastic cage like container so that every time the toilet is flushed, a quantity of water passes over the solid composition, eroding a part thereof before flushing into the toilet bowl. The toilet rim block preferably additionally includes a perfume to provide a lingering pleasant odour after every toilet flush.

### Dishwash compositions

The composition of the invention may be useful for upkeep of kitchen items e.g.. utensils and dishes which may be cleaned using a liquid dishwash compositions. The various ingredients other than the essential ingredients claimed in the present invention which may be included are anionic surfactants, non-ionic surfactants, and hydrotropes. Additionally, other ingredients like amphoteric surfactants and organic acids, may be included. Preferably, liquid dishwash composition further comprises water in an amount ranging from 5 to 99 wt%, more preferably from 15 to 80 wt%, further more preferably from 30 to 65 wt%, by weight of the composition. Preferred dishwash compositions comprise 0.1 to 5 wt% amphoteric surfactant.

Preferably, an amphoteric surfactants is selected from include alkyl amine oxides, alkyl betaines, alkyl amidopropyl betaines, alkyl sulfobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, having alkyl radicals containing from about 8 to about 22 carbon atoms, the term "alkyl" being used to include the alkyl portion of higher acyl radicals. More preferably, an amphoteric surfactant selected from alkyl amidopropyl betaines, even more preferably cocoamidopropyl betaine. Amphoteric surfactant, when included, may be present in an amount ranging preferably from 0.1 to 5 wt%, more preferably from 0.1 to 4 wt%, even more preferably from 1 to 3 wt%. Mixtures of any of the above described materials may also be used. A good combination of surfactants for use in the present invention in the form of a dishwash composition is one having 1 to 30 wt% anionic surfactant and 0.1 to 5 wt% an amphoteric surfactant.

Preferably, liquid dishwash composition further comprises one or more sequestrants which may be included in 0.1 to 5 w% by weight of the composition. A preferred sequestrant is a phosphonic acid or a salt thereof. The most preferred sequestrant is 1 -Hydroxyethylidene-1,1 - diphosphonic acid (HEDP). It is preferable that the sequestrant is added to the formulation in acid form. Optionally, liquid dishwash composition further comprises an enzyme with or without a suitable enzyme stabilizer. Preferably, viscosity of the composition may suitably range from about 200 to about 10,000 mPa.s at 25°C at a shear rate of 21 sec⁻¹. The liquid dishwash composition preferably has pH in the range from 2.0 to 8.0, more preferably from 3.5 to 7.0. The liquid dishwash composition may further comprise one or more polymers which may be included in 0.001 to 10 wt%, more preferably from 0.1 to 6 wt%, still further more preferably from 1 to 3 wt%.

Preferably, the composition may be used as is, i.e. neat, or it may be diluted before use. The extent of dilution is generally dependent on market choice. In some markets a more concentrated product is desired while in others a more dilute product is preferred. When the composition is a liquid dishwash compositions it is typically diluted with water in a weight ratio in the range of 1:1 to 1:10.

The liquid dishwash composition may optionally comprise ingredients, such as fragrance, colorant, foam boosting agents, and odor absorbing materials.

The composition is generally in liquid form. It contains high amount of water. Water is generally present in 80 to 97%, preferably 90 to 94 % by weight of the composition.

In most hard surface cleaning application, the diluted solution is applied on to the desired surface by using a brush or a mop and allowing it to dry. In certain vertical surfaces, it may be sprayed on using a hand held spray pump. In certain cases, the compositions after it is applied on to the desired surface, may be wiped off to a substantially dry condition using a dry cloth or wipe. In toilet cleaning applications, the composition is generally applied on to the toilet bowl, allowed to stay thereon for a few minutes, and then the toilet is flushed, preferably after brushing with a toilet brush.

Another preferred embodiment of the invention relates to compositions according to the invention for use as or incorporation in industrial and/or institutional products. More preferably, this embodiment of the invention relates to a composition according to the invention which is an industrial and/or an institutional product. Industrial and institutional products are for example products being marketed under professional brands, with non-limiting examples being products for industrial, institutional, janitorial, and medical cleaning, cleaning-in-place, food services, veterinary, and agricultural products.

Yet another aspect of the present invention relates to a method of removing fungus that produce DHN melanin, from a surface comprising the step of applying a composition of the present invention on to a desired surface followed by removing said composition from the surface by rinsing with water or by wiping it free of the composition.

The method may also be enabled as a two step process which comprises the steps of:
(a) Applying an aqueous solution of an alkyl substituted dihydroxy benzene wherein the alkyl group comprises 2 to 6 carbon atoms on to the desired surface; followed by
(b) cleaning the surface with an aqueous solution of one or more cationic surfactant selected from alkyl dimethyl benzylammonium chloride (ADBAC),
   alkyl diethyl benzylammonium chloride (ADEBAC), bis (C8-C18) alkyl di methyl
   quaternary ammonium chloride preferably didecyl dimethyl ammonium chloride (DDAC),
   cetyl trimethyl ammonium chloride (CTAC), cetyl pyridinium chloride (CPC), cetrimonium bromide , benzethonium chloride (BZE); and dimethyldioctadecylammonium chloride.

The above method may be utilised by the consumer by way of a product which may be a kit for removal of such fungus from a surface comprising:
(a) an aqueous solution of an alkyl substituted dihydroxy benzene wherein the alkyl group comprises 2 to 6 carbon atoms on to said surface;
(b) an aqueous solution of one or more cationic surfactant selected from
   alkyl dimethyl benzyl ammonium chloride (ADBAC),
   alkyl diethyl benzylammonium chloride (ADEBAC), bis (C8-C18) alkyl di methyl
   quaternary ammonium chloride preferably didecyl dimethyl ammonium chloride (DDAC), cetyl trimethyl ammonium chloride (CTAC), cetyl pyridinium chloride (CPC), cetrimonium bromide , benzethonium chloride (BZE); and dimethyldioctadecylammonium chloride; and
(c) instructions for use

The invention shall now be exemplified by the following non-limiting examples.

### Examples

### Example 1-2: Effect of 4-ethyl resorcinol and 4-hexyl resorcinol on inhibition of fungal melanin

### Example- 1: Fungus Aspergillus brasiliensis was tested using 1mM solution of 4-ethyl resorcinol using the following procedure.

*Aspergillus brasiliensis* culture was grown in petridishes containing potato dextrose agar and allowed to grow for 72 hours at 25 C.

Tissue culture flasks of 25 cm² was taken in enough quantities and molten PDA was added to the sides of the flasks and allowed to solidify.

Ethyl resorcinol was prepared and diluted to obtain concentrations ranging from 1mM to 100 mM. Various concentrations of ER were added to the flasks, swirled and allowed to stand for 10-15 min.

*Aspergillus* spores was suspended in 0.1% Tween 80 and the concentration of the spores was adjusted to 10⁴ cfu/ml.

1 ml of the spore suspension was added to the flasks, swirled and kept for 10-15 min.

After the contact time, the suspension was removed and the flask was incubated for 4-5 days in 25°C. The flasks was observed regularly for growth of the fungus.

For the quantification of fungal melanin, the contents of the flasks were removed to a sterile flask using a scraper.

The biomass is then treated with 1% DMSO in 0.1 N NaOH to dissolve melanin.

This solution is then filtered through a 0.22 µM filter to remove the fungal debris.

The filtrate is taken into 96 well plates and OD measured at 405 nm.

The percentage melanin is calculated over the control.

It was observed that 4-ER was capable of inhibiting fungal melanin at 79.2 % at the above described conditions.

Example -2: An experiment was conducted using 250 µM solution of 4-hexyl resorcinol using the above described procedure in Example -1.

It was observed that 4-HR was capable of inhibiting fungal melanin at 73.0% at the above described conditions.

From the data in in Example 1 and 2 above, it is clear that resorcinol compounds as claimed are effective in inhibiting fungal melanin.

### Example 3: Effect of resorcinols in reducing adhesion of fungus on surfaces

An experiment was carried out as below:
*Aspergillus brasiliensis* culture was grown in petridishes containing potato dextrose agar and allowed to grow for 72 hours at 25c.

Tissue culture flasks of 25 cm² was taken in enough quantities and molten PDA was added to the sides of the flasks and allowed to solidify.

Ethyl resorcinol was prepared and diluted to obtain concentrations ranging from 1mM to 100 mM. Various concentrations of ER were added to the flasks, swirled and allowed to stand for 10-15 min.

*Aspergillus* spores was suspended in 0.1% Tween 80 and the concentration of the spores was adjusted to 10⁴ cfu/ml.

1 ml of the spore suspension was added to the flasks, swirled and kept for 10-15 min.

After the contact time, the suspension was removed and the flask was incubated for 4-5 days in 25C. The flasks were observed regularly for growth of the fungus.

Once the fungal growth was established (5-7 days), BKC solution was added to the flasks at a concentration of 0.5 % and the flasks were kept in a shaking water bath for 24 h at room temperature.

The flasks were removed and given a gentle water rinse. The entire fungal biomass was removed from the flasks with ER treatment, where as in the control flask (with no ER treatment), the fungal biomass was intact and not removed.

It is clear from Example 3 that compositions and methods as per the present invention are capable of reducing adhesion of fungus on surfaces thereby providing a way to clean surfaces heavily contaminated with fungus.

## Claims

1. Use of an alkyl substituted dihydroxy benzene compound with the alkyl group having 2 to 6 carbon atoms for inhibition of fungal melanin in fungi that produce DHN melanin.

2. Use as claimed in claim 1 wherein the fungi is selected from one or more of Aspergillus *Brasiliensis , Aspergillus nidulans, Aspergillus niger, Alternaria alternata, Cladosporium carionii, Exophiala jeanselmei, Fonsecaea compacta, Fonsecaea pedrosoi, Hendersonula toruloidii, Phaeoannellomyces wernickii, Phialophora richardsiae, Phialophora verrucosa, Wangiella dermatitidis, and Xylohypha. Bantiana.*

3. Use of an alkyl substituted dihydroxy benzene compound with the alkyl group having 2 to 6 carbon atoms for reducing adhesion of fungus that produce DHN melanin, on surfaces.

4. A composition for removal of fungus from surfaces comprising
(i) alkyl substituted dihydroxy benzene wherein the alkyl group comprises 2 to 6 carbon atoms; and
(ii) one or more cationic surfactant selected from alkyl dimethyl benzylammonium chloride(ADBAC),
alkyl diethyl benzylammonium chloride (ADEBAC), bis (C8-C18) alkyl di methyl quaternary ammonium chloride preferably didecyl dimethyl ammonium chloride (DDAC), cetyl trimethyl ammonium chloride (CTAC), cetyl pyridinium chloride (CPC), cetrimonium bromide , benzethonium chloride (BZE); and dimethyldioctadecylammonium chloride.

5. A composition as claimed in claim 4 wherein the alkyl substituted dihydroxy benzene compound is selected from one or more of 4-ethyl resorcinol, 4-butyl resorcinol and 4-hexyl resorcinol.

6. A composition as claimed in any one of the preceding claims 4 or 5 comprising 0.05 to 4 % alkyl substituted dihydroxy benzene compound by weight of the composition.

7. A composition as claimed in any one of the preceding claims 4 to 6 comprising 0.01 to 2% cationic surfactant by weight of the composition.

8. A composition as claimed in any one of the preceding claims 4 to 7 which is a fabric conditioning composition additionally comprising 1 to 50% of a fabric softening active.

9. The composition as claimed in any one of the preceding claims 4 to 7 which is a liquid dishwash composition additionally comprising 0.1 to 5 wt% an amphoteric surfactant.

10. The composition as claimed in any one of the preceding claims 4 to 7 which is a general purpose or a toilet cleaning composition additionally comprising 0.1 to 3 wt% of a non-ionic surfactant.

11. A method of removing fungus that produce DHN melanin, from a surface comprising the step of applying the composition as claimed in any one of the preceding claims 4 to 10 on to said surface followed by removing said composition from the surface by rinsing with water or by wiping it free of said composition.

12. A method of removing fungus that produce DHN melanin, from a surface comprising the steps of:
(a) Applying an aqueous solution of an alkyl substituted dihydroxy benzene wherein the alkyl group comprises 2 to 6 carbon atoms on to said surface; followed by
(b) Cleaning said surface with an aqueous solution of one or more cationic surfactant selected from alkyl dimethyl benzylammonium chloride (ADBAC), alkyl diethyl benzylammonium chloride (ADEBAC), bis (C8-C18) alkyl di methyl quaternary ammonium chloride preferably didecyl dimethyl ammonium chloride (DDAC), cetyl trimethyl ammonium chloride (CTAC), cetyl pyridinium chloride (CPC), cetrimonium bromide , benzethonium chloride (BZE); and dimethyldioctadecylammonium chloride.

13. A kit for removal of fungus that produce DHN melanin, from a surface comprising
(a) an aqueous solution of an alkyl substituted dihydroxy benzene wherein the alkyl group comprises 2 to 6 carbon atoms on to said surface;
(b) an aqueous solution of one or more cationic surfactant selected from alkyl dimethyl benzyl ammonium chloride (ADBAC),
alkyl diethyl benzylammonium chloride (ADEBAC), bis (C8-C18) alkyl di methyl quaternary ammonium chloride preferably didecyl dimethyl ammonium chloride (DDAC), cetyl trimethyl ammonium chloride (CTAC), cetyl pyridinium chloride (CPC), cetrimonium bromide , benzethonium chloride (BZE); and dimethyldioctadecylammonium chloride; and
(c) instructions for use.

## Patentansprüche

1. Verwendung einer alkylsubstituierten Dihydroxybenzolverbindung mit einer Alkylgruppe mit 2 bis 6 Kohlenstoffatomen zur Hemmung von Pilzmelanin in Pilzen, die DHN-Melanin produzieren.

2. Verwendung, wie im Anspruch 1 beansprucht, wobei die Pilze unter einem oder mehreren der folgenden Pilze ausgewählt sind: Aspergillus Brasiliensis, Aspergillus nidulans, Aspergillus niger, Alternaria alternata, Cladosporium carionii, Exophiala jeanselmei, Fonsecaea compacta, Fonsecaea pedrosoi, Hendersonula toruloidii, Phaeoannellomyces wernickii, Phialophora richardsiae, Phialophora verrucosa, Wangiella dermatitidis und Xylohypha Bantiana.

3. Verwendung einer alkylsubstituierten Dihydroxybenzolverbindung mit der Alkylgruppe mit 2 bis 6 Kohlenstoffatomen zur Verringerung der Haftung von Pilzen, die DHN-Melanin produzieren, auf Oberflächen.

4. Zusammensetzung zur Entfernung von Pilzen von Oberflächen, umfassend
(i) alkylsubstituiertes Dihydroxybenzol, wobei die Alkylgruppe 2 bis 6 Kohlenstoffatome umfasst; und
(ii) ein oder mehrere kationische Tenside, ausgewählt unter Alkyldimethylbenzylammoniumchlorid (ADBAC), Alkyldiethylbenzylammoniumchlorid (ADEBAC), quaternärem Bis (C8-C18)-alkyldimethylammoniumchlorid, vorzugsweise Didecyldimethylammoniumchlorid (DDAC), Cetyltrimethylammoniumchlorid (CTAC), Cetylpyridiniumchlorid (CPC), Cetrimoniumbromid, Benzethoniumchlorid (BZE) und Dimethyldioctadecylammoniumchlorid.

5. Zusammensetzung, wie im Anspruch 4 beansprucht, wobei die alkylsubstituierte Dihydroxybenzolverbindung unter einer oder mehreren der folgenden Verbindungen ausgewählt ist: 4-Ethylresorcin, 4-Butylresorcin und 4-Hexylresorcin.

6. Zusammensetzung, wie in irgendeinem der vorhergehenden Ansprüche 4 oder 5 beansprucht, umfassend 0,05 bis 4 Gewichts-% der Zusammensetzung alkylsubstituierte Dihydroxybenzolverbindung.

7. Zusammensetzung, wie in irgendeinem der vorhergehenden Ansprüche 4 bis 6 beansprucht, umfassend 0,01 bis 2 Gewichts-% der Zusammensetzung kationisches Tensid.

8. Zusammensetzung, wie in irgendeinem der vorhergehenden Ansprüche 4 bis 7 beansprucht, die eine Textilkonditionierungszusammensetzung ist, die zusätzlich 1 bis 50% eines Textilweichmachers umfasst.

9. Zusammensetzung, wie in irgendeinem der vorhergehenden Ansprüche 4 bis 7 beansprucht, die eine flüssige Geschirrspülzusammensetzung darstellt, die zusätzlich 0,1 bis 5 Gew.-% eines amphoteren Tensids umfasst.

10. Zusammensetzung, wie in irgendeinem der vorhergehenden Ansprüche 4 bis 7 beansprucht, bei der es sich um eine Allzweck- oder eine Toilettenreinigungszusammensetzung handelt, die zusätzlich 0,1 bis 3 Gew.-% eines nichtionischen Tensids umfasst.

11. Verfahren zum Entfernen von Pilzen, die DHN-Melanin produzieren, von einer Oberfläche, umfassend den Schritt des Auftragens der Zusammensetzung, wie in irgendeinem der vorhergehenden Ansprüche 4 bis 10 beansprucht, auf die Oberfläche und anschließendes Entfernen der Zusammensetzung von der Oberfläche durch Abspülen mit Wasser oder durch Abwischen der Zusammensetzung.

12. Verfahren zum Entfernen von Pilzen, die DHN-Melanin produzieren, von einer Oberfläche, umfassend die Schritte:
(a) Aufbringen einer wässrigen Lösung eines alkylsubstituierten Dihydroxybenzols, wobei die Alkylgruppe 2 bis 6 Kohlenstoffatome umfasst, auf die Oberfläche; anschließend
(b) Reinigen der Oberfläche mit einer wässrigen Lösung eines oder mehrerer kationischer Tenside, ausgewählt unter Alkyldimethylbenzylammoniumchlorid (ADBAC), Alkyldiethylbenzylammoniumchlorid (ADEBAC), quaternärem Bis (C8-C18)-alkyldimethylammoniumchlorid, vorzugsweise Didecyldimethylammoniumchlorid (DDAC), Cetyltrimethylammoniumchlorid (CTAC), Cetylpyridiniumchlorid (CPC), Cetrimoniumbromid, Benzethoniumchlorid (BZE) und Dimethyldioctadecylammoniumchlorid.

13. Kit zum Entfernen von Pilzen, die DHN-Melanin produzieren, von einer Oberfläche, umfassend
(a) eine wässrige Lösung eines alkylsubstituierten Dihydroxybenzols, wobei die Alkylgruppe 2 bis 6 Kohlenstoffatome auf der Oberfläche enthält;
(b) eine wässrige Lösung eines oder mehrerer kationischer Tenside, ausgewählt unter Alkyldimethylbenzylammoniumchlorid (ADBAC), Alkyldiethylbenzylammoniumchlorid (ADEBAC), quaternärem Bis (C8-C18)-alkyldimethylammoniumchlorid, vorzugsweise Didecyldimethylammoniumchlorid (DDAC), Cetyltrimethylammoniumchlorid (CTAC), Cetylpyridiniumchlorid (CPC), Cetrimoniumbromid, Benzethoniumchlorid (BZE) und Dimethyldioctadecylammoniumchlorid; und
(c) Gebrauchsanweisungen.

## Revendications

1. Utilisation d'un composé dihydroxybenzène à substitution alkyle dont le groupe alkyle a 2 à 6 atomes de carbone, pour inhiber la mélanine fongique dans des champignons qui produisent de la DHN-mélanine.

2. Utilisation selon la revendication 1, dans laquelle le champignon est un ou plusieurs choisis parmi *Aspergillus brasiliensis, Aspergillus nidulans, Aspergillus niger, Alternaria alternata, Cladosporium carionii, Exophiala jeanselmei, Fonsecaea compacta, Fonsecaea pedrosoi, Hendersonula toruloidii, Phaeoannellomyces wernickii, Phialophora richardsiae, Phialophora verrucosa, Wangiella dermatitidis,* et *Xylohypha Bantiana.*

3. Utilisation d'un composé dihydroxybenzène à substitution alkyle dont le groupe alkyle a 2 à 6 atomes de carbone, pour inhiber l'adhésion de champignons qui produisent de la DHN-mélanine sur des surfaces.

4. Composition pour éliminer des champignons sur des surfaces, comprenant :
(i) un dihydroxybenzène à substitution alkyle dans lequel le groupe alkyle comprend 2 à 6 atomes de carbone ; et
(ii) un ou plusieurs tensioactifs cationiques choisis parmi un chlorure d'alkyldiméthylbenzylammonium (ADBAC), un chlorure d'alkyldiéthylbenzylammonium (ADEBAC), un chlorure de bis(alkyle en C₈ à C₁₈)diméthylammonium quaternaire, de préférence le chlorure de didécyldiméthylammonium (DDAC), le chlorure de cétyltriméthylammonnium (CTAC), le chlorure de cétylpyridinium (CPC), le bromure de cétrimonium, le chlorure de benzéthonium (BZE) ; et le chlorure de diméthyldioctadécylammonium.

5. Composition selon la revendication 4, dans laquelle le dihydroxybenzène à substitution alkyle est un ou plusieurs choisis parmi 4-éthylrésorcinol, le 4-butylrésorcinol et le 4-hexylrésorcinol.

6. Composition selon l'une quelconque des revendications 4 et 5, comprenant 0,05 à 4 %, en poids de la composition, de dihydroxybenzène à substitution alkyle.

7. Composition selon l'une quelconque des revendications 4 à 6, comprenant 0,01 à 2 %, en poids de la composition, de tensioactif cationique.

8. Composition selon l'une quelconque des revendications 4 à 7, qui est une composition de conditionnement des étoffes, comprenant de plus 1 à 50 % d'un agent actif assouplissant pour étoffes.

9. Composition selon l'une quelconque des revendications 4 à 7, qui est une composition liquide pour le lavage de la vaisselle, comprenant de plus 0,1 à 5 % en poids d'un tensioactif amphotère.

10. Composition selon l'une quelconque des revendications 4 à 7, qui est une composition à usage général ou pour le nettoyage des toilettes, comprenant de plus 0,1 à 3 % en poids d'un tensioactif non-ionique.

11. Procédé d'élimination de champignons qui produisent de la DHN-mélanine sur une surface, comprenant l'étape d'application de la composition selon l'une quelconque des revendications 4 à 10 sur ladite surface, suivie de l'élimination de ladite composition sur la surface par rinçage à l'eau ou par essuyage jusqu'à ce qu'elle soit exempte de la composition.

12. Procédé d'élimination de champignons qui produisent de la DHN-mélanine sur une surface, comprenant les étapes de :
(a) application d'une solution aqueuse d'un dihydroxybenzène à substitution alkyle dans lequel le groupe alkyle comprend 2 à 6 atomes de carbone sur ladite surface ; puis
(b) nettoyage de ladite surface avec une solution aqueuse d'un ou plusieurs tensioactifs cationiques choisis parmi un chlorure d'alkyldiméthylbenzylammonium (ADBAC), un chlorure d'alkyldiéthylbenzylammonium (ADEBAC), un chlorure de bis(alkyle en C₈ à C₁₈)diméthylammonium quaternaire, de préférence le chlorure de didécyldiméthylammonium (DDAC), le chlorure de cétyltriméthylammonnium (CTAC), le chlorure de cétylpyridinium (CPC), le bromure de cétrimonium, le chlorure de benzéthonium (BZE) ; et le chlorure de diméthyldioctadécylammonium.

13. Kit pour éliminer des champignons qui produisent de la DHN-mélamine sur une surface, comprenant
(a) une solution aqueuse d'un dihydroxybenzène à substitution alkyle dans lequel le groupe alkyle comprend 2 à 6 atomes de carbone sur ladite surface ;
(b) une solution aqueuse d'un ou plusieurs tensioactifs cationiques choisis parmi un chlorure d'alkyldiméthylbenzylammonium (ADBAC), un chlorure d'alkyldiéthylbenzylammonium (ADEBAC), un chlorure de bis(alkyle en C₈ à C₁₈)diméthylammonium quaternaire, de préférence le chlorure de didécyldiméthylammonium (DDAC), le chlorure de cétyltriméthylammonnium (CTAC), le chlorure de cétylpyridinium (CPC), le bromure de cétrimonium, le chlorure de benzéthonium (BZE) ; et le chlorure de diméthyldioctadécylammonium ; et
(c) des instructions pour l'utilisation.
